# EUROPÄISCHE PATENTSCHRIFT

(11) **EP 0 450 169 B1**
(45) Veröffentlichungstag und Bekanntmachung des Hinweises auf die Patenterteilung: **11.05.1994**
(21) Anmeldenummer: 90123934.3
(22) Anmeldetag: 12.12.1990
(51) Int. Cl.: C07D 207/277

(54) **Verfahren zur Herstellung von 3-Carboxamido-5-vinyl-2-pyrrolidon**
Process for the preparation of 3-carboxamido-5-vinyl-2-pyrrolidone
Procédé pour la préparation de carboxamido-3-vinyl-5-pyrrolidone-2

(30) Priorität: 03.04.1990 DE 4010709
(43) Veröffentlichungstag der Anmeldung: 09.10.1991
(73) Patentinhaber: HÜLS AKTIENGESELLSCHAFT, 45764 Marl (DE)
(72) Erfinder: Steffen, Klaus-Dieter, Dr., W-5202 Hennef 1 (DE)

(56) Entgegenhaltungen:
- DE-A- 2 902 438

## Beschreibung

Die Erfindung betrifft ein Verfahren zur Herstellung von 3-Carboxamido-5-vinyl-2-pyrrolidon aus 2-Vinylcyclopropan-1,1-dicarbonsäuredialkylestern und Ammoniak.
Gemäß der Erfindung wird 2-Vinylcyclopropan-1,1-dicarbonsäuredialkylester als Ausgangsstoff eingesetzt und die Reaktion nach Zugabe von Hydrochinon als Polymerisationsinhibitor und von Alkali-bzw. Erdalkalialkoholat als Katalysator durchgeführt wird. 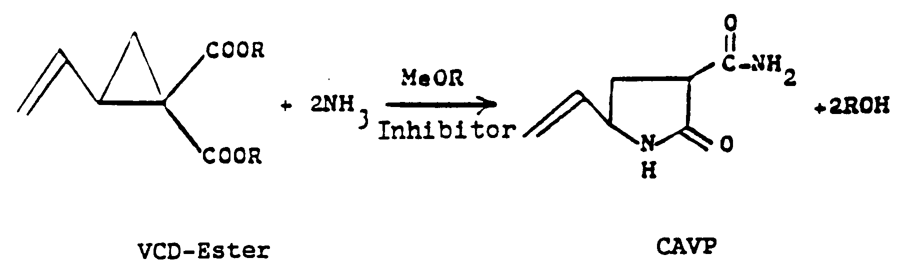
Me: Alkalimetall; R: -Alkyl C₁ bis C₅

Die Herstellung und Verwendung dieses Pyrrolidon-Derivats ist bereits in der DE-OS 2 902 438 (US-PS 873 272) beschrieben. Es werden hier keinerlei Angaben über Ausbeuten und Reinheiten der dargestellten Verbindung gemacht. Der im Beispiel 3 des erwähnten Patents angegebene Schmelzpunkt von 215°C ist falsch, ein Hinweis dafür, daß die Synthese dieser Substanz nach den dort gemachten Angaben nur mit schlechten Reinheiten durchführbar ist (siehe Vergleichsbeispiel).
Die Pyrrolidon-Derivate werden zur Herstellung von 4-Amino-hexen-5-säure (Vinyl-GABA) verwendet und dann als irreversible Inhibitoren der γ-Aminobuttersäure-Transaminase eingesetzt.

Die Ausgangsverbindung 2-Vinylcyclopropyl-1,1-dicarbonsäuredialkylester, 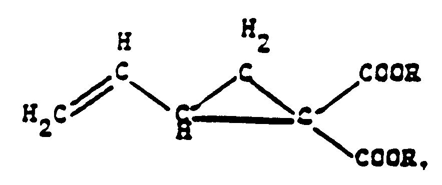
in der R einen C₁- bis C₅-Alkylrest - bevorzugt Methyl- oder Äthyl-Rest - bedeuten, kann nach R.W. Kierstead u.a., J. Chem. Soc. 1952, Seiten 3610 bis 3616, aus einem Malonester H₂C (COOR)₂ und trans-1,4-Dibrombuten-2 hergestellt werden. Die Reste R können gleich oder verschieden sein.
Diese Verbindung ist stets durch 1,1-Dicarbonsäuredialkylester-cylcopenten-3 verunreinigt, insbesondere dann, wenn das eingesetzte 1,4-Dibrombuten-2 ein Gemisch des cis- und trans-Isomeren darstellt. Außerdem neigt diese Vinylcyclopropyl-Verbindung zur Polymerisation.

Es bestand also die Aufgabe, die Reaktion so zu steuern, daß 2-Vinylcyclopropyl-1,1-dicarbonsäuredialkylester als verunreinigtes Rohprodukt eingesetzt werden kann und trotzdem das Zielprodukt in hoher Reinheit und guter Ausbeute erhalten wird.

Gegenstand der Erfindung ist daher das Verfahren nach den Patentansprüchen.

Die Aufgabe konnte insbesondere dadurch gelöst werden, daß man die Reaktion nach Zugabe von Hydrochinon als Polymerisationsihibitor in Mengen von 0,1 bis 2,0 g, bevorzugt 0,4 bis 0,8 g pro Mol Vinylcyclopropan-dicarbonsäureester, und eines Katalysators wie Alkalialkoholat in Mengen von 0,2 bis 3%, bevorzugt von 0,5 bis 1,5%, bezogen auf Vinylcyclopropan-dicarbonsäuredialkylester, durchführt. Das Alkoholat wird zweckmäßigerweise als Lösung in dem betreffenden Alkohol des vinylcyclopropan-dicarbonsäuredialkylesters eingesetzt. Zur Anwendung kommen die Alkalialkoholate, bevorzugt die Natrium- und Kaliumalkoholate, ggf. auch Erdalkalialkoholate. Dadurch ist es möglich, die Vinylcyclopropyl-dicarbonsäuredialkylester auch als "Rohprodukt", d.h. als unreinen Ausgangsstoff mit Reinheiten von 50 bis 98%, einzusetzen. Nebenreaktionen und Zersetzungen können unterdrückt werden (z.B. zu Ammoncarbonat), so daß das Zielprodukt 3-Carboxamido-5-vinyl-2-pyrrolidon (CAVP) in ausgezeichneten Reinheiten von über 99% und guten Ausbeuten um 65% erhalten wird.

Die Ausbeute an CAVP bezieht sich auf das Isomeren-Gemisch, da die Verbindung zwei asymmetrische C-Atome besitzt und ist auf umgesetzten Vinylcyclopropyl-dicarbonsäuredialkylsäureester berechnet.

Die Reaktion wird in der Regel bei 110 bis 130°C in 15 bis 30 Stunden bei einem Druck von 10 bis 15 bar durchgeführt. Als Lösungsmittel dienen bevorzugt die niederen Alkohole wie Methanol, Äthanol.
Ammoniakgas kann im Überschuß von 1:3 (Mole VCD-Ester: NH₃) eingesetzt werden. Der NH₃-Überschuß wird am Reaktionsende in das Lösungsmittel des Folgeansatzes entspannt, so daß in diesen nachfolgenden Ansatz nur etwas mehr als die stöchiometrische Menge NH₃ eindosiert werden muß.
Das nachfolgende Vergleichsbeispiel und die anschließenden Versuche mögen dieses Verfahren erläutern.

### Vergleichsbeispiel analog DE-OS 2 902 438, Beisp.3

In einen 1-1-Glaskolben werden 600 ml Formamid und 112,5 g 2-Vinylcyclopropan-1,1-dicarbonsäuredimethylester (81,7%-ig an VCDM, entsprechend 0,5 Mol) eingefüllt und unter Rühren auf 128°C aufgeheizt. Nach Durchleiten von 270 g NH₃-Gas innerhalb von 20 h wird angekühlt und das gesamte Formamid unter Vakuum (16 bis 2 mbar) abdestilliert. Der feste Rückstand wird aus ca. 200 ml Methanol umkristallisiert.
Es resultieren 26,1 g 3-Carboxamido-5-vinyl-2-pyrrolidon (33,8% d.Th.) mit einem Schmelzpunkt von 191°C. Durch Einengen des methanolischen Filtrats resultieren weitere 6 g stark verunreinigtes Zielprodukt.

### Beispiel 1

In einem mit Rührer versehenen, ca. 1,5 l fassenden Glasautoklaven, der durch einen Ölthermostaten beheizbar ist, werden der Reihe nach eingefüllt: 112 g 2-Vinylcyclopropan-1,1-dicarbonsäuredimethylester (VCDM 82,4%-ig an VCDM, entsprechend 0,5 Mol), 400 ml Methanol, 0,2 g Hydrochinon und 1,12 g Natriummethylat als methanolische Lösung (1% bezüglich auf VCDM). Nach dem Verschließen des Autoklaven werden 50 g NH₃-Gas aus einer Druckflasche aufgedrückt und die Reaktion in 22 h bei 120°C unter einem Druck von ca. 13 bar durchgeführt. Nach Reaktionsende wird abgekühlt, entspannt, der Autoklav entleert und mit Methanol gespült. Die gesamte methanolische Suspension ist bei Normaldruck bis zur beginnenden Kristallisation einzuengen, dann abzukühlen, und anschließend sind die 3-Carboxamido-5-vinyl-2-pyrrolidon-2-Kristalle zu isolieren und zu trocknen.

| | |
|---|---|
| Ausbeute: | 49,9 g (64,7% d.Th.) |
| Schmelzpunkt: | 191-193°C |
| Reinheit (G.C.): | 99,5% |

### Beispiel 2

Unter denselben Bedingungen und in derselben Apparatur, wie in Beispiel 1 beschrieben, werden der Reihe nach eingefüllt: 137 g 2-Vinylcyclopropan-1, 1-dicarbonsäurediäthylester (VCDE, 77,7%ig entsprechend 0,5 Mol), 500 ml Äthanol, 0,5 g Hydrochinon, 5 g Natriumäthanolat als 20 %-ige äthanolische Lösung und anschließend 52 g NH₃-Gas aufgedrückt. Nach 22 h Reaktionszeit bei 123°C und ca. 13 bar Druck wird abgekühlt, entspannt und der Inhalt mit Äthanol in einen Glaskolben entleert. Äthanol wird bis zur beginnenden Kristallisation abdestilliert, der Ansatz abgekühlt und das Kristallisat abfiltriert, mit Äthanol gewaschen und getrocknet. Das äthanolische Filtrat wird aufdestilliert und nicht umgesetztes VCDE zurückgewonnen: 29 g, was einem Umsatz auf eingesetztes VCDE von 73% entspricht.

| | |
|---|---|
| Ausbeute an CAVP: | 36 g (64,1% d.Th.) |
| Schmelzpunkt: | 192-194°C |
| Reinheit (G.C.): | 99,9% |

## Patentansprüche

1. Verfahren zur Herstellung von 3-Carboxamido-5-vinyl-2-pyrrolidon aus 2-Vinylcyclopropan-1,1-dicarbonsäuredialkylester und Ammoniak, **dadurch gekennzeichnet**, daß die Reaktion mit 2-Vinylcyclopropan-1,1-dicarbonsäuredialkylester als Ausgangsstoff und nach Zugabe von Hydrochinon als Polymerisationsinhibitor sowie in Gegenwart eines Alkali- oder Erdalkalialkoholats als Katalysator bei 110 bis 130°C und einem Druck von 10 bis 15 bar durchgeführt wird.

2. Verfahren nach Anspruch 1, **dadurch gekennzeichnet**, daß man Hydrochinon in Mengen von 0,1 bis 2,0 g pro Mol Vinyl-cyclopropan-dicarbonsäuredialkylester zusetzt.

3. Verfahren nach Anspruch 1 und 2, **dadurch gekennzeichnet**, daß Alkalialkoholat in Mengen von 0,2 bis 3,0%, bezogen auf Vinylcyclopropan-dicarbonsäuredialkylester zugegeben wird.

4. Verfahren nach Anspruch 1 bis 3, **dadurch gekennzeichnet**, daß man den 2-Vinylcyclopropan-1,1-dicarbonsäuredialkylester in einer Reinheit von 50 bis 98% verwendet.

## Claims

1. Method for the preparation of 3-carboxamido-5-vinyl-2-pyrrolidone from 2-vinylcyclopropan-1,1-dicarboxylic acid dialkylesters and ammonia, characterised in that the reaction is carried out at 110 to 130°C and a pressure of 10 to 15 bar with 2-vinylcyclopropan-1,1-dicarboxylic acid dialkylester as starting material and after addition of hydroquinone as polymerisation inhibitor, as well as in the presence of an alkali or alkaline earth alcoholate as catalyst.

2. Method according to claim 1, characterised in that hydroquinone is added in amounts of 0.1 to 2.0 gram per mol. of vinylcyclopropanedicarboxylic acid dialkylester.

3. Method according to claim 1 and 2, characterised in that alkali alcoholate is supplied in amounts of 0.2 to 3.0 % related to vinylcyclopropanedicarboxylic acid dialkylester.

4. Process according to claim 1 to 3, characterised in that 2-vinylcyclopropan-1,1-dicarboxylic acid dialkylester is used in a purity of 50 to 98%.

## Revendications

1. Procédé pour la préparation de la 3-carboxamido-5-vinyl-2-pyrrolidone à partir d'un ester dialkylique d'acide 2-vinylcyclopropane-1,1-dicarboxylique et d'ammoniac, caractérisé en ce qu'on effectue la réaction avec un ester dialkylique de l'acide 2-vinylcyclopropane-1,1-dicarboxylique comme matière de départ et après addition de l'hydroquinone comme inhibiteur de polymérisation, ainsi qu'en présence d'un alcoolate alcalin ou alcalinoterreux comme catalyseur, à 110 jusqu'à 130°C et sous une pression de 10 à 15 bars.

2. Procédé selon la revendication 1, caractérisé en ce qu'on ajoute l'hydroquinone en quantités de 0,1 à 2,0 g par mole de l'ester dialkylique de l'acide vinylcyclopropane-dicarboxylique.

3. Procédé selon les revendications 1 et 2, caractérisé en ce qu'on ajoute l'alcoolate alcalin en quantités de 0,2 à 3,0 % par rapport à l'ester dialkylique de l'acide vinylcyclopropane-dicarboxylique.

4. Procédé selon l'une des revendications 1 à 3, caractérisé en ce qu'on utilise l'ester dialkylique de l'acide 2-vinylcyclopropane-1,1-dicarboxylique ayant une pureté de 50 à 98 %.
